# EUROPEAN PATENT SPECIFICATION

(11) **EP 1 478 321 B1**
(45) Date of publication and mention of the grant of the patent: **06.07.2005**
(21) Application number: 02707106.7
(22) Date of filing: 28.02.2002
(51) Int. Cl.: A61K 6/00

(54) **METHOD FOR REPRODUCING IMAGES ON COSMETIC COMPOSITIONS**
VERFAHREN ZUM REPRODUZIEREN EINES BILDES AUF EIN KOSMETIKUM
PROCEDE DE REPRODUCTION D'IMAGES SUR DES COMPOSITIONS COSMETIQUES

(43) Date of publication of application: 24.11.2004
(73) Proprietor: Gamma Croma S.p.A., 26010 Vaiano Cremasco (IT)
(72) Inventor: ANCOROTTI, Renato, I-26013 Crema (IT); PRIORE, Romualdo, I-20129 Milano (IT)
(74) Representative: Marietti, Andrea
(86) International application number: PCT/IT2002/000124
(87) International publication number: WO 2003/072069

(56) References cited:
- EP-A- 1 238 816
- DE-A- 19 730 887
- ES-A- 2 027 585
- FR-A- 2 759 941
- US-A- 5 312 240
- US-A1- 2001 012 055
- PATENT ABSTRACTS OF JAPAN vol. 012, no. 005 (C-467), 8 January 1988 (1988-01-08) & JP 62 164610 A (SHISEIDO CO LTD), 21 July 1987 (1987-07-21)

## Description

### TECHNICAL FIELD

The present invention relates to a method for the reproduction of images on a substrate consisting of a cosmetic composition in the form of powder, paste or a solid, such as the compositions used, for example, for making face powder, foundation cream, eye shadow, or other products for makeup.

In what follows, the term "cosmetic compositions in powder form" will basically be taken to mean those compositions in compacted powder form that can be contained in a container or case - such as a powder compact or, to use the French term, "godet" - for example eye shadow, face powder or rouge; the term "compositions in the form of paste" will be taken to mean those compositions in the form of a "waxy" paste, such as lipstick, eye powder, foundation cream (but also some types of eye shadow and rouge); whilst the term "solid compositions" will be taken to mean new-concept products for makeup devised by the present applicant and described in the Italian patent application IT MI 2001 A 002841.

### PRIOR ART

It constitutes prior art to reproduce, on the top surface of a cosmetic composition, or starting therefrom, an image which, by being rendered visible from outside thanks to containers that are at least partially transparent, enables the possible buyer to recognize the product and/or to be induced into purchasing the said product. The shape or aesthetic appearance of cosmetic compositions contributes to the success of the product on the market.

In particular, it constitutes known art to set cosmetic compositions for makeup consisting of porous surfaces, such as compacted powder or materials in the form of "waxy" paste or solid materials, inside containers or "godets" which have an open top surface. The outer surface, or the superficial layers, of the cosmetic composition set facing the open side of the godet may constitute a substrate on which a desired image may be made, such as an ornamental pattern.

The Italian patent IT 1 237 771 teaches how to make a cosmetic product consisting of stacked layers of powder of different colours. By engraving the said cosmetic product to different depths it is possible to make coloured figures or compositions that can be perceived when the outer surface of the product is viewed. Engraving of the product can be carried out by a "digging" operation, prior to the possible compression of the powder.

Although the above method for making patterns of aesthetic interest on the cosmetic product enables pleasant chromatic compositions to be obtained, it is not suitable for reproducing figures and proves particularly complex and difficult to implement. In fact, for the reproduction of other than elementary figures, given also the consistency of the cosmetic product, which may for instance be porous or viscous, it would be necessary to perform extremely precise "digging" operations which would entail sophisticated mechanisms of regulation and control of the tool designed for carrying out the said operation.

In the French patent FR 2 759 941 it has likewise been proposed to decorate a cosmetic composition made of compacted powder or paste by depositing ink on its outermost surface. By using an appropriately modified inkjet printer and inks with non-toxic pigments or dyes that are acceptable for cosmetic use, it is possible to reproduce any figure on the substrate consisting of the outer surface of the composition.

The process proposed in the above-mentioned published patent FR 2 759 941 is simple to use and extremely versatile. However, the nonhomogeneous absorption and the unforseeable diffusion of the ink within many cosmetic compositions frequently leads to results that are far from satisfactory, on account of the presence of smudges, as well as on account of the poor clarity of the reproduction of the image. Furthermore, the addition to the aforesaid cosmetic compositions of extraneous pigments contained in the ink renders this technique applicable only to a limited number of products and may lead to the degradation of the physical and/or chromatic characteristics of the cosmetic composition itself.

From DE-A-19730887 a method for creating an image on a soap or a food product is known.

A purpose of the present invention is therefore to provide a method for making an image on a substrate consisting of a cosmetic composition in the form of powder, paste or a solid that will not present the drawbacks of the prior art.

A further purpose of the present invention is to provide a method that will enable reproduction of complex images on the surface or on the superficial layers of a powder, paste or solid cosmetic product in a fast and precise way.

Yet another purpose of the present invention is to propose the use of an apparatus for engraving or marking objects as a tool for reproducing images on cosmetic products.

Yet a further purpose of the present invention is to provide a new cosmetic product that presents at least one outer surface on which there is reproduced, in an extremely accurate way, an image chosen by the manufacturer.

### DESCRIPTION OF THE INVENTION

The above and other purposes are achieved by the method for making an image on a substrate consisting of a cosmetic composition in the form of powder, paste or a solid, according to the first independent claim and the subsequent dependent claims, by the use of an apparatus for making figures on a substrate for representing, on said substrate, an image consisting of a cosmetic composition in the form of powder, paste or a solid, according to Claim 12 and the subsequent dependent claims, and by the cosmetic composition according to the independent Claim 19 and the subsequent dependent claims.

The method for making an image on a substrate consisting of a cosmetic composition in the form of powder, paste or a solid, according to the present invention, comprises the steps of:
- subjecting at least parts of the aforesaid substrate to irradiation by a laser beam; and
- displacing the laser beam with respect to the substrate, or vice versa, in order to trace the desired image on the substrate.

This method enables, basically according to the characteristics of the cosmetic composition and of the laser beam used, to bring about chromatic variations on the outer surface of the cosmetic composition and/or engravings of said composition in order to reproduce any image that the manufacturer may intend to create.

Furthermore, according to a preferential aspect of the present invention, the method also involves the steps of:
- storing an image to be reproduced on the cosmetic product in digital form;
- generating, in an automatic way, the commands for operating the laser beam and/or commands for displacement of the laser with respect to the substrate according to the image stored, in order to enable tracing of the image on the substrate; and
- executing said commands.

Furthermore, preferably, these steps are preceded by a step of scanning and digitization of an image placed on a plate-like or film-like support, such as a photograph or slide. This enables reproduction, basically on any surface of a cosmetic composition, of a pre-chosen image, in an automated and simple way.

According to another aspect of the present invention, for implementation of the aforesaid method the use is envisaged of an apparatus for the formation of images on a substrate by means of a laser beam, in which said substrate is a cosmetic composition made of powder, paste or a solid. The laser apparatus may be equipped with means for scanning images from photographs or slides (transparencies) and then enabling immediate transfer of images onto the surface of the cosmetic product.

The present invention likewise regards a cosmetic product, preferably for makeup, that presents a visible surface on which an image is traced by means of a laser beam. The said image may be created by chromatic variation of the surface of the cosmetic composition or by engraving of the superficial layers of the latter, according to the type of cosmetic composition and the type of laser used.

### BRIEF DESCRIPTION OF THE DRAWINGS

In what follows, some embodiments of the present invention will be described, purely by way of non-limiting examples, with the aid of the following figures, in which:
Figure 1 is a block diagram illustrating a method according to a preferential aspect of the present invention;
Figure 2 is a block diagram illustrating another method according to a particularly advantageous aspect of the present invention;
Figure 3 is a functional diagram of apparatus for implementation of the method according to a preferred aspect of the present invention;
Figure 4 is a schematic plan view, partially cut-away, of apparatus for implementation of a method according to the present invention; and
Figure 5 is a plan-view image of an embodiment of a product for makeup according to the present invention.

### DETAILED DESSCRIPTION OF SOME EMBODIMENTS OF THE INVENTION

The method according to the present invention basically consists in applying a coherent beam of monochromatic light generated by a laser apparatus on a substrate consisting of a cosmetic composition. The precise displacement of the laser beam with respect to the substrate, given the extremely reduced diameter of the beam (which may be of a few tenths of a millimetre) and the possibility of accurate adjustment of the operating parameters of the laser make it possible to trace lines or to highlight particular areas of the substrate so as to reproduce thereon one or more pre-defined images.

The action of the laser beam on the surface of a cosmetic product may basically produce two effects according to the characteristics of operation of the laser (power supplied, exposure time, etc.) and according to the physical state and state of aggregation of the cosmetic: inducing a chromatic surface variation of the product and/or engraving the superficial layers of the product. In either case, the action of the laser on the surface of a cosmetic product enables reproduction of images that may be seen by the user.

Application of the above method, which is known as laser marking, to cosmetic products is new and has proved capable of producing surprising results, as will be clarified in what follows.

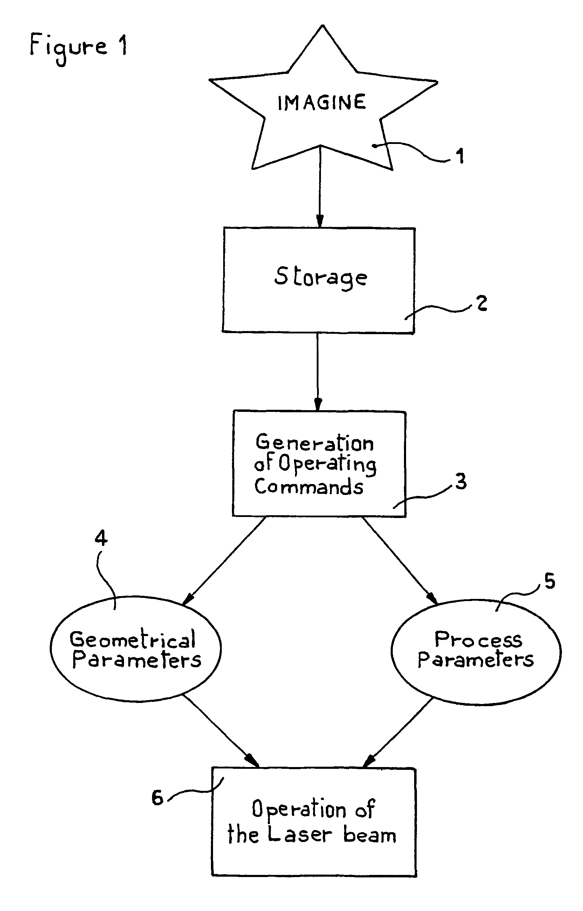

Figure 1 illustrates a particular embodiment of the method according to the present invention which presupposes the use of an automatic apparatus for laser marking. The method envisages the steps of:
1. creation of an image 1, for example by means of a computer medium;
2. storage 2 of the image, preferably in a digital format, in processing means;
3. automatic generation 3, by means of algorithms known to the art, of commands for driving a laser beam; and
4. operation 6 of the laser beam with respect to a surface of a cosmetic product, to execute the commands generated in step 3, for reproducing the image 1.

In particular, the commands for operating the laser beam, which are distinctive of the laser apparatus used, usually consist of instructions containing geometrical parameters regarding the displacement paths of the laser beam with respect to the substrate or vice versa and instructions with process parameters, such as speed of displacement of the laser beam and/or of the substrate, the power supplied, the stay time of the laser beam on a given surface, etc.

The set of instructions generated in step 3 of the method illustrated enables reproduction of the image 1 on the surface of the cosmetic product.

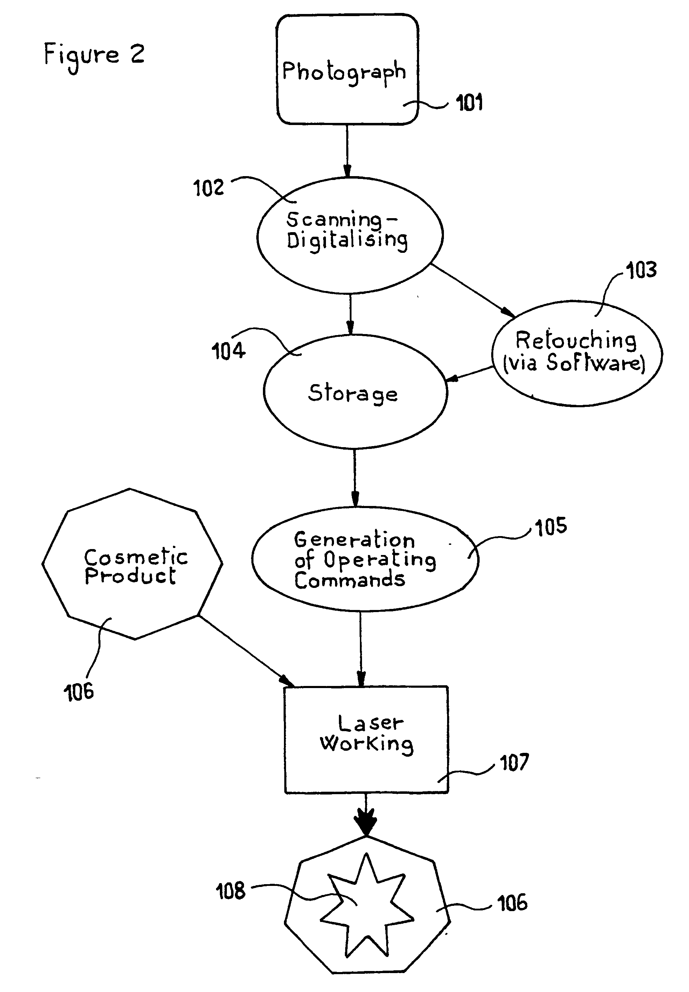

Figure 2 illustrates another preferred method for the reproduction of images on cosmetic products according to the present invention. This method envisages that the creation of the image to be reproduced takes place by acquisition, via a scanner, of figures from a plate-like medium (photographs) or film-like medium (slides), and that, following upon acquisition of the image and its digitization and storage on processing media, there may be envisaged a step of "touching-up" of the image by means of software tools.

In greater detail, the method illustrated presupposes the use of processing means and comprises the steps of:
- scanning 102 of images and their digitization starting from a photograph 101, or other material medium (drawings on paper, slides, etc.);
- storage 102 of the digitized image, preferably reproduced in 256 shades of grey, and its possible modification by means of software tools;
- generation 105 of the commands for operation of a laser beam;
- preparation of a cosmetic product 106 of the type made of powder, paste or a solid; and
- processing 107 by means of laser beam of the external surface of the cosmetic product 106, for reproduction of the image 108 on the worked surface of the cosmetic product.

The cosmetic product 106, which may, for example, be a product for makeup made of compacted powder, such as a face powder, or of paste, such as a foundation cream, is then placed under the laser beam so that the latter will induce chromatic modifications and/or engrave the surface of the product 106 in such a way as to reproduce the image 108. In the case of cosmetic products 106 made of powder or paste, these are set directly in the final container ("godet") prior to their processing under the laser beam.

As an alternative to step 102 of scanning and digitization of the image 108 on a material medium 101 it is possible envisage a step (not illustrated) of creation of the image 108 by means of computer tools for design.

According to a preferred aspect of the method according to the present invention, as already described, by exploiting the presence of light-sensitive and/or heat-sensitive pigments in the cosmetic product 106, the laser beam is operated in such a way as to induce a chromatic variation on the most superficial layer of the cosmetic product 106. The process parameters to which the laser beam is subjected during processing determine the intensity of the chromatic variation of the surface of the cosmetic product (i.e., it is possible to form shadows and areas of light and dark on the product itself) and determine the degree of penetration of the chromatic variation within the cosmetic product. It has surprisingly been found that the said laser-marking technique applied to cosmetic products enables reproduction of images with great accuracy, in various shades, on the surface of said cosmetic products, without the innermost layers of the product being affected ,and hence without any substantial modifications to the colouring of the product, except for its most superficial layer (having a depth estimated at a few microns).

Consequently, this enables reproduction of any image on the external surface of a cosmetic product in a faithful way, for instance set on a photograph, without thereby modifying the characteristics of the cosmetic product itself. In addition, the use of laser makes it possible to subject basically any cosmetic product to the said process without incurring in poor results due to the particular nature of the cosmetic product.

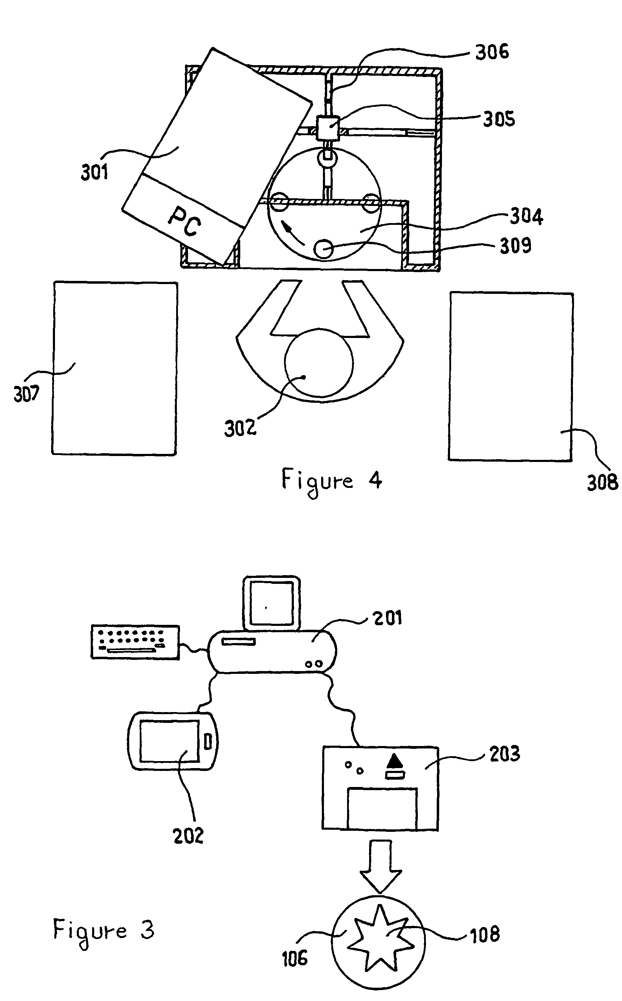

With reference now to Figure 3, an apparatus is described for creating figures on a substrate by means of laser (laser marking), suitable for implementation of the method according to the present invention. Falling within the intents of the present invention is likewise the protection of the use of an apparatus for laser marking on substrates consisting of cosmetic products made of powder, paste or solids.

The apparatus, according to a particular aspect of the present invention, comprises a processing control unit 201, which is operatively connected to a device 203 for marking a substrate 204 by means of a laser beam, and a device 202 for the scanning of images.

For implementation of the method described with reference to Figure 2, the image present on a photograph 101 scanned and digitized (step 102) by the device 202, consisting for example of a commercial scanner, is stored (step 104) on the mass-storage device of the computer 201, such as a hard disk. The image stored can then be modified (step 103) by means of a program for graphical retouching which runs on the computer 201 - the said program, for example, reproducing the image in shades of grey - and then sending it on for reading to a special program (driver) designed for converting the graphical information of the stored image into geometrical and process commands for the device 203 (step 105). The process of conversion of the graphical information into commands for driving a device equipped with a laser beam forms part of the known art and is altogether similar to the processes commonly used, for instance, for driving the printing head of commercial laser printers.

Execution of the above commands (step 107) by the processing device 203 enables formation of the image 108 on a substrate 106 consisting of a cosmetic composition made of powder, paste or a solid.

Figure 4 is a schematic illustration, partially in cut-away view, of a workstation equipped with an apparatus for reproduction of a pre-defined image on a makeup product by means of a laser beam.

In particular, this is an apparatus that comprises processing means 301 designed for the storage of the image to be reproduced and for control of a device 303 for laser marking, represented in Figure 4 partially in cutaway view.

The processing means 301, consisting of any commercial computer, can be connected to a scanner for digitizing images present on photographs or slides.

The device 303 comprises a worktable 304 and a processing head 305 which carries the output lens for the laser beam (not represented) and which is mounted in a translatable way along at least one axis with respect to the worktable 304. The processing head 305 is driven in translation by actuators 306, for instance ones comprising electric stepper motors.

Alternatively, in an embodiment not illustrated herein, it is possible to envisage the presence of actuators for displacing the table 304 with respect to the processing head 305.

Advantageously, in the embodiment illustrated in Figure 4, the worktable 304 is circular and is rendered rotatable with respect to an axis that passes through its centre by actuator means (not illustrated). The products 309 that are to be set beneath the head 305 are arranged radially around the circumference of the table, in such a way that rotation of the table 304 carries the products 309 in a sequential way underneath the processing head 305.

For processing, the operator 302 has available a tray 307, on which it is possible to arrange the cosmetic products that have not yet undergone processing by the laser beam of the head 305, for instance rouge contained in the "godets", and a tray 308 for the products 309 on which the image has been formed by the laser. During the production cycle, the operator 302 can mount the products that are to undergo processing on the circular worktable 305 and can remove the products 309 that have already been processed, whilst a product is simultaneously undergoing processing beneath the head 305. This enables minimization of dead time during production and ensures high productivity.

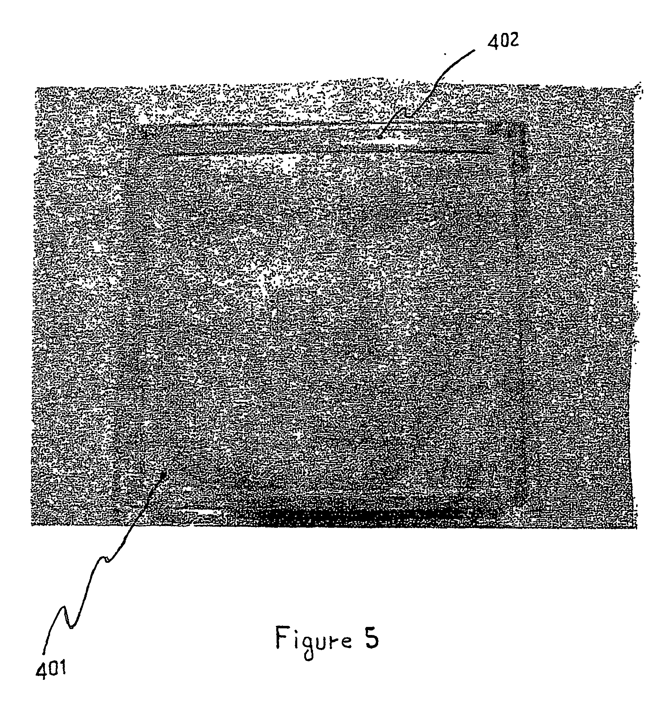

The products 309 obtained by laser processing, such as the product represented in Figure 5, present clear images thanks to the chromatic nuances produced by the laser beam, also in the case of cosmetic compositions with dark colouring. In addition, the precision with which the laser traces the images that are to be reproduced on the makeup product makes it possible to subject also cosmetic products of extremely small dimensions to the method according to present invention.

### EXAMPLE

Various cosmetic compositions, of the type made of compacted powder and paste, were subjected to a commercial apparatus for the marking of objects.

After an image to be reproduced underwent scanning, by means of a commercial scanner with a scanning resolution of 300 dpi (dots per inch), the said image was stored in 256 shades of grey on a computer.

The computer was operatively connected to a commercially available marking device and was equipped with software for driving a laser-marking head. The marking device comprised a gas laser of the CO₂ type, capable of supplying up to 300 W of power with a wavelength of 10.6 micron.

After various specimens had undergone processing, they revealed a substantial lack of effect of laser marking on the physical and chromatic characteristics of the cosmetic composition, as well as a clear reproduction of the image only on the most superficial layer of the specimen.

It has moreover been proposed to use a marking device equipped with a Nd:YAG or Er:YAG laser.

Figure 5 illustrates a specimen consisting of face powder 401 obtained by means of powder compacted inside a powder compact (or "godet") 402 and then subjected to processing with a CO₂ laser. The 256 shades of grey with which a photograph representing a woman's face was stored were reproduced thanks to the action of the laser, which, according to the power supplied and/or the time of exposure which were variable from point to point on the surface of the face powder 401, rendered different areas of the superficial portion of the product more or less dark, without these chromatic variations reaching any great depth and thus altering the characteristics of the face powder 401 itself.

## Claims

1. A method for creating an image (1, 108) on a substrate consisting of a cosmetic composition (106) *for makeup* made of powder, paste or a solid, comprising the steps of:
a. subjecting at least parts of said substrate to irradiation by means of a laser beam; and
b. displacing said laser beam with respect to said substrate, or vice versa, for tracing said image (1, 108) on the substrate;
**characterised in that** said image (1, 108) is reproduce in various shades, by means of chromatic variations induced by the laser beam, on the superficial layer of said substrate.

2. The method according to Claim 1, **characterized in that** it comprises the steps of:
a. storing the image (1, 108) that is to be created in digital form;
b. generating a set of commands for operating said laser beam and/or displacing said laser beam with respect to said substrate, or vice versa, according to the image stored, said commands enabling tracing of the image (1, 108) on the substrate: and
c. operating the laser beam and displacing said laser beam with respect to said substrate, or vice versa, in order to execute said commands.

3. The method according to Claim 2, in which said commands comprise instructions for displacing the laser beam with respect to the substrate, or vice versa, and/or instructions for varying the operating parameters of the laser.

4. The method according to Claim 2 or Claim 3, **characterized in that** it comprises, before said step of storing the image (1, 108) to be reproduced in digital form, a step of scanning of the image (1, 108) placed on a plate-like or film-like medium.

5. The method according to Claim 4, in which said image (1, 108) is a photograph.

6. The method according to Claim 2 or Claim 3, **characterized in that** it comprises, before said step of storing the image (1, 108) to be reproduced in digital form, a step of creation of the design or pattern in digital form.

7. The method according to any one of Claims 2 to 6, in which said image (1, 108) in digital form in stored in a plurality of shades of grey.

8. The method according to any one of the foregoing claims, in which said substrate is a makeup product chosen from among a rouge, a face powder, a lipstick, a compact foundation cream, and an eye shadow.

9. The method according to any one of the foregoing claims, in which said substrate is placed in a container prior to the step of subjecting at least parts of said substrate to laser radiation.

10. Use of an apparatus for forming, by means of laser, figures on a substrate for representing an image (1, 108) on a substrate consisting of a cosmetic composition *for makeup* made of powder or paste or a solid, wherein said image (1, 108) is obtained in various shades by chromatic variations of its superficial layer due to the action of the laser beam.

11. Use according to Claim 10. In which said apparatus comprises a device for scanning an image (1, 108) set on a plate-like or film-like medium.

12. Use according to Claim 11, in which said image (1, 108) is a photograph.

13. Use according to any one of Claims 10 to 12, in which said beam originates from a gas laser apparatus, preferably of the CO2 type, or from a laser apparatus of the Nd : YAG or Er : YAG type.

14. Use according to any one of Claims 10 to 13, in which said laser beam is sustained by a processing head (305), and said apparatus comprises actuator means (306) for displacement of the head (305) with respect to the substrate an/or vice versa.

15. Use according to any one of Claims 10 to 14, in which said apparatus comprises a circular woktable (304) for supporting, around its circumference, one or more of said substrates.

16. Use according to Claim 14 or Claim 15, in which said apparatus comprises computer-processing means (101) for storing the image (1, 108) and for automatic control of said laser beam and/or of said actuator means (306) and/or of said worktable (304).

17. A cosmetic composition *for makeup* made of powder or paste or a solid, **characterized in that** its superficial layer reproduces an image (1, 108) in various shades, said image having been obtained by chromatic variation of its superficial layer due to the action of the laser beam.

18. The composition according to Claim 17, in which said composition is a makeup product chosen from among a rouge, a face powder, a lipstick, a compact foundation cream, and an eye shadow.

## Patentansprüche

1. Verfahren zum Reproduzieren eines Bildes (1, 108) auf ein aus einer kosmetischen Zusammensetzung (106) bestehendes Substrat für Make-up in Pulver-. Pasten- oder in fester Form, umfassend die folgenden Schritte:
a. Aussetzen einer Strahlung mittels eines Laserstrahls von zumindest Teilen des genannten Substrats, sowie
b. Verschieben des genannten Laserstrahls gegenüber dem genannten Substrat oder umgekehrt, um das genannte Bild (1, 108) auf dem Substrat nachzuzeichnen,
**dadurch gekennzeichnet, dass** das genannte Bild (1, 108) in unterschiedlichen Schattierungen durch von dem Laserstrahl induzierte Farbänderungen auf der Oberflächenschicht des genannten Substrats reproduziert wird.

2. Verfahren nach Anspruch 1, **dadurch gekennzeichnet, dass** es die folgenden Schritte umfasst:
a. Speichern des Bildes (1, 108), das erzeugt werden soll, in digitaler Form;
b. Generieren eines Befehlssatzes zum Betrieb des genannten Laserstrahls und/oder zum Verschieben des genannten Laserstrahls gegenüber dem genannten Substrat, oder umgekehrt, gemäß dem gespeicherten Bild, wobei die Befehle es ermöglichen, das Bild (1, 108) auf dem Substrat nachzuzeichnen; und
c. Betrelben des Laserstrahls und Verschieben des genannten Laserstrahls gegenüber dem genannten Substrat, oder umgekehrt, um die genannten Befehle auszuführen.

3. Verfahren nach Anspruch 2, **dadurch gekennzeichnet, dass** die genannten Befehle Anweisungen zum Verschieben des Laserstrahls gegenüber dem Substrat, oder umgekehrt, umfassen und/oder Anweisungen zum Variieren der Betriebsparameter des Lasers.

4. Verfahren nach Anspruch 2 oder Anspruch 3, **dadurch gekennzeichnet, dass** es vor dem Schritt des Speichems des zu reproduzierenden Bildes (1, 108) in digitaler Form einen Schritt des Abtastens des Bildes (1, 108) umfasst, das auf einem platten- oder filmartigen Medium aufliegt.

5. Verfahren nach Anspruch 4, bei dem das Bild (1,108) eine Fotografie ist.

6. Verfahren nach Anspruch 2 oder Anspruch 3, **dadurch gekennzeichnet, dass** es vor dem Schritt des Speichems in digitaler Form des zu reproduzierenden Bildes (1, 108) einen Schritt des Erzeugens der Zeichnung oder des Musters in digitaler Form umfasst.

7. Verfahren nach irgendeinem der Ansprüche 2 bis 6, bei dem das genannte Bild (1, 108) in digitaler Form in einer Mehrzahl von Graustufen gespeichert wird.

8. Verfahren nach irgendeinem der vorhergehenden Ansprüche, bei dem das genannte Substrat ein Make-up-Erzeugnis ist, das aus Rouge, Gesichtspuder, Lippenstift, Kompakt-Grundierungscreme und Lidschatten ausgewählt wird.

9. Verfahren nach irgendeinem der vorhergehenden Ansprüche, bei dem das genannte Substrat in einen Behälter verbracht wird, bevor der Schritt des Bestrahlens von zumindest Teilen des genannten Substrats ausgeführt wird.

10. Verwendung einer Vorrichtung zum Formen mittels eines Laserstrahls von Figuren auf einem Substrat zum Darstellen eines Bildes (1, 108) auf einem Substrat, das aus einer kosmetischen Zusammensetzung für Make-up in Pulver-, Pasten- oder fester Form besteht, wobei aufgrund der Wirkung des Laserstrahls das genannte Bild (1, 108) durch Farbänderungen seiner Oberflächenschicht In verschiedenen Schattierungen erzeugt wird.

11. Verwendung nach Anspruch 10, bei der die genannte Vorrichtung ein Gerät zum Abtasten eines Bildes (1, 108) umfasst, das auf einem platten- oder filmartigen Medium aufliegt.

12. Verwendung nach Anspruch 11, bei der das genannte Bild (1, 108) eine Fotografie ist.

13. Verwendung nach irgendeinem der Ansprüche 10 bis 12, bei der der genannte Strahl von einer Gaslaservorrichtung stammt, vorzugsweise des CO₂-Typs, oder von einer Laservorrichtung des Nd:YAG- oder Er:YAG-Typs.

14. Verwendung nach irgendeinem der Ansprüche 10 bis 13, bei der der Laserstrahl durch einen Bearbeitungskopf (305) aufrechterhalten wird und die genannte Vorrichtung Betätigungsmittel (306) umfasst, um den Kopf (305) gegenüber dem genannten Substrat und/oder umgekehrt zu verschieben.

15. Verwendung nach irgendeinem der Ansprüche 10 bis 14, bei der die genannte Vorrichtung einen runden Arbeitstisch (304) umfasst, um ein oder mehrere der genannten Substrate auf seinem Umfang zu tragen.

16. Verwendung nach Anspruch 14 oder Anspruch 15, bei der die genannte Vorrichtung Computer unterstützte Mittel (101) zum Speichern des Bildes (1, 108) und zum automatischen Steuern des genannten Laserstrahls und/oder der genannten Betätigungsmittel (306) und/oder des genannten Arbeitstisches (304) umfasst.

17. Kosmetische Zusammensetzung für Make-up in Pulver-, Pasten- oder fester Form, **dadurch gekennzeichnet, dass** seine Oberflächenschicht ein Bild (1, 108) in verschiedenen Schattierungen wiedergibt, wobei das genannte Bild durch Farbänderungen seiner Oberflächenschicht durch die Einwirkung des Laserstrahls erzeugt worden ist.

18. Zusammensetzung nach Anspruch 17, bei dem die genannte Zusammensetzung ein Make-up-Erzeugnis ist, das aus Rouge, Gesichtspuder, Lippenstift, Kompakt-Grundierungscreme und Lidschatten ausgewählt wird.

## Revendications

1. Procédé pour créer une image (1, 108) sur un substrat constitué par une composition cosmétique (106) pour maquillage préparée à partir d'une poudre, d'une pâte ou d'une matière solide, comprenant les étapes consistant à :
a. soumettre au moins des parties dudit substrat à un rayonnement au moyen d'un faisceau laser ; et
b. déplacer ledit faisceau laser par rapport audit substrat, ou *vice versa,* pour tracer ladite image (1, 108) sur le substrat ;
**caractérisé en ce que** ladite image (1, 108) est reproduite dans diverses nuances, au moyen de variations chromatiques induites par le faisceau laser, sur la couche superficielle dudit substrat.

2. Procédé selon la revendication 1, **caractérisé en ce qu'**il comprend les étapes consistant à :
a. mémoriser l'image (1, 108) qui doit être créée sous forme numérique ;
b. générer un ensemble de commandes pour actionner ledit faisceau laser et/ou déplacer ledit faisceau laser par rapport audit substrat, ou *vice versa*, selon l'image mémorisée, lesdites commandes permettant le traçage de l'image (1, 108) sur le substrat ; et
c. actionner le faisceau laser et déplacer ledit faisceau laser par rapport audit substrat, ou *vice versa,* afin d'exécuter lesdites commandes.

3. Procédé selon la revendication 2, dans lequel lesdites commandes comprennent des instructions pour déplacer le faisceau laser par rapport au substrat, ou *vice versa,* et/ou des instructions pour modifier les paramètres de fonctionnement du laser.

4. Procédé selon la revendication 2 ou la revendication 3, **caractérisé en ce qu'**il comprend, avant ladite étape de mémorisation de l'image (1, 108) à reproduire sous forme numérique, une étape consistant à balayer l'image (1, 108) placée sur un support en forme de plaque ou en forme de film.

5. Procédé selon la revendication 4, dans lequel ladite image (1, 108) est une photographie.

6. Procédé selon la revendication 2 ou la revendication 3, **caractérisé en ce qu'**il comprend, avant ladite étape de mémorisation de l'image (1, 108) à reproduire sous forme numérique, une étape de création du dessin ou du motif sous forme numérique.

7. Procédé selon l'une quelconque des revendications 2 à 6, dans lequel ladite image (1, 108) sous forme numérique est mémorisée en une pluralité de nuances de gris.

8. Procédé selon l'une quelconque des revendications précédentes, dans lequel ledit substrat est un produit de maquillage choisi parmi un fard à joues, une poudre pour le visage, un rouge à lèvres, un fond de teint compact et une ombre à paupières.

9. Procédé selon l'une quelconque des revendications précédentes, dans lequel ledit substrat est placé dans un récipient avant l'étape consistant à soumettre au moins des parties dudit substrat à un rayonnement laser.

10. Utilisation d'un appareil pour former, au moyen d'un laser, des figures sur un substrat pour représenter une image (1, 108) sur un substrat constitué par une composition cosmétique pour maquillage préparée à partir d'une poudre, une pâte ou d'une matière solide, dans laquelle ladite image (1, 108) est obtenue dans diverses nuances par variations chromatiques de sa couche superficielle en raison de l'action du faisceau laser.

11. Utilisation selon la revendication 10, dans laquelle ledit appareil comprend un dispositif pour balayer une image (1, 108) fixée sur un support en forme de plaque ou en forme de film.

12. Utilisation selon la revendication 11, dans laquelle ladite image (1, 108) est une photographie.

13. Utilisation selon l'une quelconque des revendications 10 à 12, dans laquelle ledit faisceau provient d'un appareil laser à gaz, de préférence du type CO₂, ou d'un appareil laser du type Nd:YAG ou Er:YAG.

14. Utilisation selon l'une quelconque des revendications 10 à 13, dans laquelle ledit faisceau laser est entretenu par une tête de traitement (305) ; et ledit appareil comprend des moyens d'actionnement (306) pour déplacer la tête (305) par rapport au substrat et/ou vice versa.

15. Utilisation selon l'une quelconque des revendications 10 à 14, dans laquelle ledit appareil comprend une table de travail (304) circulaire pour supporter, sur sa circonférence, un ou plusieurs desdits substrats.

16. Utilisation selon la revendication 14 au la revendication 15, dans laquelle ledit appareil comprend des moyens de traitement informatique (401) pour mémoriser l'image (1, 108) et pour contrôler automatiquement ledit faisceau laser et/ou lesdits moyens d'actionnement (306) et/ou ladite table de travail (304).

17. Composition cosmétique pour maquillage préparée à partir d'une poudre, d'une pâte ou d'une matière solide, **caractérisée en ce que** sa couche superficielle reproduit une image (1, 108) dans diverses nuances, ladite image étant obtenue par variation chromatique de sa couche superficielle en raison de l'action du faisceau laser.

18. Composition selon la revendication 17, dans laquelle ladite composition est un produit de maquillage choisi parmi un fard à joues, une poudre pour le visage, un rouge à lèvres, un fond de teint compact et une ombre à paupières.
